(19) Europäisches Patentamt
European Patent Office
Office européen des brevets

(11) Veröffentlichungsnummer: **0 008 715**
**B1**

(12) # EUROPÄISCHE PATENTSCHRIFT

(45) Veröffentlichungstag der Patentschrift: **25.11.81**

(51) Int. Cl.[3]: **C 07 C 125/06,** C 07 C 127/19, A 61 K 31/325, A 61 K 31/17

(21) Anmeldenummer: **79102967.1**

(22) Anmeldetag: **16.08.79**

(54) **Phenyläthanolamine zur Verwendung bei der Bekämpfung von entzündlichen Zuständen.**

(30) Priorität: **07.09.78 DE 2838923**

(43) Veröffentlichungstag der Anmeldung: **19.03.80 Patentblatt 80/6**

(45) Bekanntmachung des Hinweises auf die Patenterteilung: **25.11.81 Patentblatt 81/47**

(84) Benannte Vertragsstaaten: **BE CH DE FR GB IT LU NL SE**

(56) Entgegenhaltungen:
**DE-A-2 261 914**
**DE-A-2 710 997**

(73) Patentinhaber: **Dr. Karl Thomae GmbH, Postfach 720, D-7950 Biberach (Riss) (DE)**

(72) Erfinder: **Engelhardt, Günther, Dr., Unterer Bühl 18, D-7950 Biberach 1 (DE)**
Erfinder: **Keck, Johannes, Dr. Dipl.-Chem., Talfeldstrasse 27, D-7950 Biberach 1 (DE)**
Erfinder: **Krüger, Gerd, Dr. Dipl.-Chem., Ginsterhalde 30, D-7950 Biberach 1 (DE)**
Erfinder: **Noll, Klaus, Dr. Dipl.-Chem., Im Schönblick 3, D-7951 Warthausen 1 (DE)**
Erfinder: **Pieper, Helmut, Dr. Dipl.-Chem., Kapellenweg 5, D-7950 Biberach 1 (DE)**
Erfinder: **Zimmermann, Rainer, Dr., Amriswilstrasse 7, D-7950-Biberach (DE)**

**EP 0 008 715 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Phenyläthanolamine zur Verwendung bei der Bekämpfung von entzündlichen Zuständen

Aus der Literatur ist bekannt, daß Phenyläthanolamine und deren Säureadditionssalze $\beta_2$-mimetische und/oder $\beta_1$-blockierende Eigenschaften aufweisen. So werden in der BE-PS 808 743 Phenyläthanolamine der allgemeinen Formel Ia

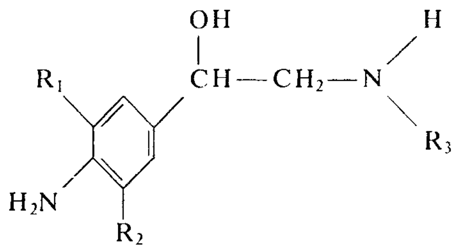

(Ia)

und in der nicht vorveröffentlichten BE-PS 864 834 Phenyläthanolamine der allgemeinen Formel Ib

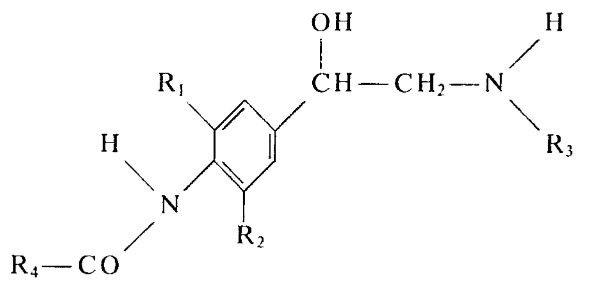

(Ib)

und deren physiologisch verträgliche Säureadditionssalze beschrieben, die insbesondere $\beta_2$-mimetische und/oder $\beta_1$-blockierende Wirkungen aufweisen.

In den obigen allgemeinen Formeln Ia und Ib bedeutet

$R_1$ ein Wasserstoff-, Fluor-, Chlor-, Brom- oder Jodatom oder die Cyangruppe,
$R_2$ ein Fluoratom, die Cyan-, Trifluormethyl- oder Nitrogruppe oder eine Alkylgruppe mit 1 bis 4 Kohlenstoffatomen,
$R_3$ eine Cycloalkyl- oder Alkylgruppe mit jeweils 3 bis 5 Kohlenstoffatomen und
$R_4$ eine Alkoxygruppe mit 1 bis 5 Kohlenstoffatomen, eine Alkenyloxygruppe mit 2 bis 5 Kohlenstoffatomen, eine Aryloxygruppe mit 6 bis 10 Kohlenstoffatomen, eine Aralkoxygruppe mit 7 bis 11 Kohlenstoffatomen oder eine Aminogruppe, die durch eine Alkylgruppe mit 1 bis 5 Kohlenstoffatomen, eine Alkenylgruppe mit 2 bis 5 Kohlenstoffatomen, eine Arylgruppe mit 6 bis 10 Kohlenstoffatomen und/oder eine Aralkylgruppe mit 7 bis 11 Kohlenstoffatomen mono- oder disubstituiert sein kann.

Unter den bei der Definition der Reste $R_3$ und $R_4$ erwähnten Bedeutungen kommt für

$R_3$ die der Isopropyl-, sec.Butyl-, Isobutyl-, tert.Butyl-, sec.Pentyl-, Isopentyl-, tert.Pentyl-, Cyclopropyl-, Cyclobutyl- oder Cyclopentylgruppe und für
$R_4$ die der Methoxy-, Äthoxy-, n-Propoxy-, Isopropoxy-, n-Butoxy-, sec.Butoxy-, Isobutoxy-, tert.Butoxy-, n-Pentyloxy-, Isopentyloxy-, tert.Pentyloxy-, Phenoxy-, Naphthyloxy-, Benzyloxy-, Phenyläthyloxy-, Allyloxy-, Butenyloxy-, Pentenyloxy-, Amino-, Methylamino-, Äthylamino-, n-Propylamino-, Isopropylamino-, n-Butylamino-, sec.Butylamino-, Isobutylamino-, tert.Butylamino-, n-Pentylamino-, Dimethylamino-, Diäthylamino-, Di-n-propylamino-, Diisopropylamino-, Di-n-butylamino-, Di-sec.butylamino-, Diisobutylamino-, Di-tert.butylamino-, Di-n-pentylamino-, Phenylamino-, Naphthylamino-, Benzylamino-, Phenyläthylamino-, Methyläthylamino-, Methylbenzylamino-, Methyl-n-propylamino-, Methylisopropylamino-, Methyl-n-butylamino-, Methylbenzylamino-, Methyl-n-propylamino-, Methylisopropylamino-, Methyl-n-butylamino-, Äthyl-propylamino-, Äthyl-isopropylamino-, Diphenylamino-, Methyl-phenylamino-, Äthylphenylamino-, Isopropyl-phenylamino-, Allylamino-, Diallylamino-, n-Butenylamino- oder n-Pentenylaminogruppe in Betracht.

Es wurde nun überraschenderweise gefunden, daß die Verbindungen der allgemeinen Formel Ib

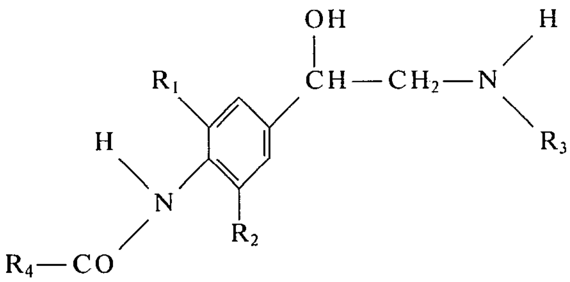

(Ib)

in der
$R_1$ bis $R_4$ wie eingangs definiert sind, und deren physiologisch verträgliche Säureadditionssalze wertvolle anti-rheumatische bzw. antiphlogistische Wirkungen aufweisen.

Beispielsweise wurde die Verbindung

A = 1-(4-Äthoxycarbonylamino-3-cyan-5-fluor-phenyl)-2-tert.butyl-amino-äthanol-hydrochlorid

auf ihre Hemmwirkung gegenüber der Freisetzung lyosomaler Enzyme ($\beta$-Glucuronidase dient als Leitenzym) aus humanen, polymorphkernigen Leukocyten (PMN) unter der Zymosan-Phagocytose untersucht. Nach Weissmann spielt die Freisetzung lyosomaler Enzyme im Rahmen der Phagocytose von Immunkomplexen eine wesentliche Rolle für die »self perpetuation« bei der rheumatoiden Arthritis.

Die Prüfung erfolgte in Anlehnung an die Methode von WEISSMANN et al. [J. Exp. Med. 134, 149s—165s (1971)]. Puffersystem und Inkubationszeiten wurden abgeändert, die Zymosanpartikel- und PMN-Zahl standardisiert. Aus den durch die Prüfsubstanz gegenüber der Zymosankontrolle erhaltenen Hemmeffekten wurde nach linearer Regressionsanalyse nach LINDER (Stat. Methoden, 4. Aufl., pp. 148—162, Birkhäuser, Basel, 1964) eine $EK_{20}$ mit den Vertrauensgrenzen nach FIELLER [Quart. J. Pharm. Pharmacol. 17, 117—123 (1944)] als die Endkonzentration der Prüfsubstanz angegeben, die eine 20%ige Hemmung der während der Zymosanphagocytose erfolgenden Freisetzung lysosomaler $\beta$-Glucuronidase aus humanen polymorphkernigen Leukocyten in vitro bewirkte.

Über die bei der Prüfung erhobenen Befunde gibt die nachfolgende Tabelle Auskunft. Die Substanz A erweist sich unter in-vitro-Bedingungen als ein wesentlich stärkerer Hemmer der Freisetzung lysosomaler Enzyme aus menschlichen polymorphkernigen Leukocyten als Terbutalinsulfat (ca. 18 mal stärker), Substanz B der nachfolgenden Tabelle. Terbutalinsulfat ist als das Sulfat des 2-tert.Butylamino-1-(3,4-dihydroxyphenyl)-äthanols chemisch mit der Substanz A verwandt.

Aufgrund dieser starken Aktivität am menschlichen Leukocyten und der im Vergleich zu anderen $\beta$-Mimetica sehr lange anhaltenden Wirkung in vivo, sowie der sehr guten enteralen Resorption dürfte die Substanz A für die Hemmung des spezifischen entzündlichen Geschehens beim Rheumapatienten wesentlich besser geeignet sein als andere $\beta$-Mimetica.

Tabelle:

Wirkung auf die unter der Zymosan-Phagocytose erfolgende Freisetzung lysomaler $\beta$-Glucuronidase aus humanen polymorphkernigen Leukocyten

| Substanz | $n_1$ | $n_2$ | Bereich der geprüften Endkonzentration in mol/l | $EK_{20}$ mit Vertrauensgrenzen in mol/l | in ng/ml |
|---|---|---|---|---|---|
| B | 4 | 103 | $10^{-8}$–$10^{-5}$ | $2{,}4 \times 10^{-6}$ | 658,3 |
| | | | | $(1{,}1–7{,}3 \times 10^{-6})$ | (301,7–2002,4) |
| A | 3 | 147 | $10^{-8}$–$10^{-6}$ | $1{,}3 \times 10^{-7}$ | 46,8 |
| | | | | $(0{,}58–3{,}1 \times 10^{-7})$ | (20,8–111,6) |

$n_1$ = Zahl der geprüften Konzentrationen.
$n_2$ = Zahl der geprüften Proben.

2. Akute Toxizität

Die akute Toxizität wurde an Mäusen beiderlei Geschlechts mit einem mittleren Gewicht von 20 g nach i. v. Applikation bestimmt. Aus dem Prozentsatz der Tiere, die nach den verschiedenen Dosen innerhalb von 14 Tagen verstarben, wurde nach Litchfield und Wilcoxon [J. Pharmacol. exp. Ther. 96, 99 (1949)] die $LD_{50}$ berechnet:

| Substanz | $LD_{50}$ mg/kg i. v. |
|---|---|
| A | 97,5 |

Die Verbindungen der allgemeinen Formel I und deren physiologisch verträgliche Salze mit anorganischen oder organischen Säuren eignen sich daher zur Bekämpfung von entzündlichen Zuständen und somit zur Behandlung der rheumatoiden Arthritis. Hierzu lassen sie sich, gegebenenfalls in Kombination mit anderen Wirksubstanzen, in die üblichen pharmazeutischen Zubereitungsformen wie Tabletten, Dragées, Lösungen, Sprays, Ampullen, Salben, Cremen, Suspensionen oder Suppositorien einarbeiten. Die Einzeldosis beträgt hierbei am Menschen 5 – 100 µg, vorzugsweise jedoch 10 – 25 µg, 2 bis 4 × täglich.

Die nachfolgenden Beispiele sollen die Erfindung näher erläutern:

Beispiel A

1-(4-Äthoxycarbonylamino-3-chlor-5-fluor-phenyl)-2-tert.butylamino-äthanol-hydrochlorid

In eine Lösung von 3,7 g Selendioxid in 30 ml Dioxan und 1 ml Wasser werden bei 60° C unter Rühren 8,7 g 4'-Äthoxycarbonylamino-3'-chlor-5'-fluor-acetophenon prtionsweise eingetragen. Anschließend wird 4 Stunden lang auf Rückflußtemperatur erhitzt. Zu der so dargestellten Lösung von 4'-Äthoxycarbonylamino-3'-chlor-5'-fluor-phenylglyoxal werden nach Abkühlen und unter Außenkühlung mit Eis 4,1 ml tert.-Butylamin getropft. Nach beendeter Zugabe verdünnt man mit 350 ml Äthanol und filtriert vom Ungelösten ab. Die das rohe 4'-Äthoxycarbonylamino-3'-chlor-5'-fluor-phenyl-glyoxyliden-tert.butylamin enthaltende Lösung wird unter Rühren und Kühlen mit Eis portionsweise mit 5 g Natriumborhydrid versetzt und über Nacht bei Raumtemperatur stehen gelassen. Anschließend zerstört man überschüssiges Natriumborhydrid mit Aceton, versetzt mit Wasser und extrahiert mit Chloroform. Die Chloroform-Lösung wird mit Wasser gewaschen, mit Natriumsulfat getrocknet, nach Zusatz von Aktivkohle kurz aufgekocht, filtriert und im Vakuum zur Trockne eingedampft. Der feste Rückstand von 1-(4-Äthoxycarbonylamino-3-chlor-5-fluor-phenyl)-2-tert.butylamino-äthanol wird in Isopropanol aufgenommen und mit ätherischer Salzsäure bis pH 4 angesäuert. Nach Zugabe von Äther tritt Kristallisation ein. Die Kristalle werden abgesaugt und mit Äther gewaschen.
Schmelzpunkt: 192 – 193° C.

Beispiel B

1-(4-Äthoxycarbonylamino-5-brom-3-methyl-phenyl)-2-tert.butylamino-äthanol

12 g 4'-Äthoxycarbonylamino-5'-brom-3'-methyl-acetophenon werden in 200 ml Chloroform gelöst und zum Sieden erhitzt. In die siedende Lösung tropft man 6,5 g Brom, das unter Bromwasserstoffentwicklung schnell verbraucht wird. Danach wird das jetzt in der Lösung vorhandene 4'-Äthoxycarbonylamino-5',2-dibrom-3'-methyl-acetophenon durch Zugabe von 14,6 g tert.Butylamin und 30minütiger kochen in 4'-Äthoxycarbonylamino-5'-brom-3'-methyl-2-tert.butylamino-acetophenon überführt. Man engt im Vakuum zur Trockne ein, nimmt den Rückstand in 50 ml Methanol und 20 ml Wasser auf und reduziert die Ketogruppe durch Zugabe einer Lösung von 3,8 g Natriumborhydrid in 20 ml Wasser, wobei gleichzeitig der pH-Wert des Reaktionsgemisches durch Zugabe von verdünnter Salzsäure zwischen 6 und 8 gehalten wird. Nach Beendigung der Reduktion wird das Methanol im Vakuum abdestilliert, der Rückstand mit Wasser verdünnt, mit Ammoniak deutlich alkalisch gestellt und mit Chloroform extrahiert. Die organische Phase wird abgetrennt, getrocknet und zur Trockne eingeengt. Der Rückstand wird über eine Kieselsäule gereinigt (Elutionsmittel: Chloroform/Methanol 2 : 1), das Eluat eingeengt, der Rückstand in Äthanol gelöst und mit ätherischer Salzsäure versetzt. Man erhält 1-(4-Äthoxy-carbonylamino-5-brom-3-methylphenyl)-2-tert.butylamino-äthanol-hydrochlorid.
Schmelzpunkt: 212 – 214° C (Zers.).

Analog Beispiel A oder B werden folgende Verbindungen hergestellt:

1-(4-Äthoxycarbonylamino-3-chlor-5-trifluormethyl-phenyl)-2-tert.butylamino-äthanol
Schmelzpunkt: 168–170° C (Zers.)

1-(4-Äthoxycarbonylamino-3-brom-5-fluor-phenyl)-2-isopropylamino-äthanol-hydrochlorid
Schmelzpunkt: 180–182° C

1-(4-Äthoxycarbonylamino-3-brom-5-fluor-phenyl)-2-tert.butylamino-äthanol-hydrochlorid
Schmelzpunkt: 197–198° C (Zers.)

1-(4-Äthoxycarbonylamino-3-fluor-phenyl)-2-tert.butylamino-äthanol-hydrochlorid
Schmelzpunkt: 235–236° C

1-(4-Äthoxycarbonylamino-3-cyan-5-fluor-phenyl)-2-tert.butylamino-äthanol-hydrochlorid
Schmelzpunkt: 198–200° C (Zers.)

1-(4-Äthoxycarbonylamino-3-nitro-phenyl)-2-tert.butylamino-äthanol-hydrochlorid
Schmelzpunkt: 189–190° C (Zers.)

1-(4-Äthoxycarbonylamino-3-fluor-5-jod-phenyl)-2-cyclopropylamin-äthanol
Schmelzpunkt: 127–130° C

1-[3-Fluor-5-jod-4-(3-methyl-ureido)-phenyl]-2-tert.butylamino-äthanol-hydrochlorid
Schmelzpunkt: Sintern ab 115° C (amorphe Substanz)
Massenspektrum ($C_{14}H_{21}N_3O_2FJ$):
Molpeak der Base: Gef.: 409 Ber.: 409,25

1-[3-Fluor-5-jod-4-(3-methyl-ureido)-phenyl]-2-cyclopropylamino-äthanol-hydrochlorid
Schmelzpunkt: 167–170° C

1-(3-Cyan-5-fluor-4-isobutyloxycarbonylamino-phenyl)-2-tert.butylamino-äthanol-hydrochlorid
(Das überschüssige Natriumborhydrid wurde mit verdünnter Salzsäure zerstört)
Schmelzpunkt: 189–191° C

1-(4-Benzyloxycarbonylamino-3-fluor-5-jod-phenyl)-2-cyclopropylamino-äthanol
(Das überschüssige Natriumborhydrid wurde mit verdünnter Salzsäure zerstört)
Schmelzpunkt: 135–137° C

1-(4-Allyloxycarbonylamino-3-fluor-5-jod-phenyl)-2-cyclopropylamino-äthanol
(Das überschüssige Natriumborhydrid wurde mit verdünnter Salzsäure zerstört)
Schmelzpunkt: 122–123° C

1-(3-Fluor-4-isobutyloxycarbonylamino-5-jod-phenyl)-2-cyclopropylamino-äthanol
(Das überschüssige Natriumborhydrid wurde mit verdünnter Salzsäure zerstört)
Schmelzpunkt: 126–128° C

1-[3-Cyan-4-(3-tert.butyl-ureido)-phenyl]-2-tert.butylaminoäthanol
Schmelzpunkt: 105–110° C (Zers.)
Massenspektrum ($C_{18}H_{28}N_4O_2$)
Molpeak der Base: Gef.: 332 Ber.: 332,45

Das als Ausgangsprodukt benötigte 4'-(3-tert.Butyl-ureido)-2-brom-3'-cyan-acetophenon wurde durch Umsetzung von 4'-Phenoxycarbonylamino-2-brom-3'-cyan-acetophenon und tert.Butylamin bei Raumtemperatur hergestellt.

1-(4-Äthoxycarbonylamino-3-cyan-phenyl)-2-isopropylamino-äthanol
Schmelzpunkt: 112–115° C

1-(4-Äthoxycarbonylamino-3-cyan-phenyl)-2-tert.butylamino-äthanol
Schmelzpunkt: 78–82° C

Beispiel 1

Tabletten mit 10 γ 1-(4-Äthoxycarbonylamino-3-cyan-5-fluor-phenyl)-2-tert.butylamino-äthanol-hydrochlorid

Zusammensetzung:

1 Tablette enthält:

| | |
|---|---|
| Wirksubstanz | 0,01 mg |
| Milchzucker | 82,49 mg |
| Kartoffelstärke | 33,00 mg |
| Polyvinylpyrrolidon | 4,00 mg |
| Magnesiumstearat | 0,50 mg |
| | 120,00 mg |

Herstellungsverfahren

Die Wirksubstanz und PVP werden in Äthanol gelöst. Die Mischung von Milchzucker und Kartoffelstärke wird mit der Wirkstoff-/Granulierlösung gleichmäßig befeuchtet. Die Feuchtsiebung erfolgt mit 1,5 mm-Maschenweite. Anschließend wird bei 50° C getrocknet und die Trockensiebung mit 1,0 mm-Maschenweite vorgenommen. Das so erhaltene Granulat wird mit Magnesiumstearat gemischt und zu Tabletten verpreßt.

| | |
|---|---|
| Tablettengewicht: | 120 mg |
| Stempel: | 7 mm, flach |

Beispiel 2

Dragées mit 5 γ 1-(4-Äthoxycarbonylamino-3-cyan-5-fluor-phenyl)-2-tert.butylamino-äthanol-hydrochlorid

Zusammensetzung:

1 Dragéekern enthält:

| | |
|---|---|
| Wirksubstanz | 0,005 mm |
| Milchzucker | 82,495 mg |
| Kartoffelstärke | 33,000 mg |
| Polyvinylpyrrolidon | 4,000 mg |
| Magnesiumstearat | 0,500 mg |
| | 120,000 mg |

Herstellungsverfahren

Dragéekerne analog Tabletten Beispiel 1.

| | |
|---|---|
| Kerngewicht: | 120 mg |
| Stempel: | 7 mm, gewölbt |

Die Kerne werden nach bekanntem Verfahren mit einer Hülle überzogen, die im wesentlichen aus Zucker und Talkum besteht. Die fertigen Dragées werden mit Bienenwachs poliert.

| | |
|---|---|
| Dragéegewicht: | 200,0 mg |

## Beispiel 3

Gelatine-Steckkapseln mit 10γ 1-(4-Äthoxycarbonylamino-3-cyan-5-fluor-phenyl)-2-tert.butylamino-äthanol-hydrochlorid

Zusammensetzung:

1 Kapsel enthält:

| | |
|---|---|
| Wirksubstanz | 0,01 mg |
| Milchzucker | 59,99 mg |
| Maisstärke | 60,00 mg |
| | 120,00 mg |

Herstellungsverfahren

Die Wirksubstanz wird mit Milchzucker und Maisstärke intensiv gemischt und in Gelatine-Steckkapseln geeigneter Größe abgefüllt.

Kapselfüllung: 120,0 mg

## Beispiel 4

Ampullen mit 10 γ 1-(4-Äthoxycarbonylamino-3-cyan-5-fluor-phenyl)-2-tert.butylamino-äthanol-hydrochlorid

Zusammensetzung:

1 Ampulle enthält:

| | | |
|---|---|---|
| Wirksubstanz | | 0,01 mg |
| Zitronensäure | | 2,5 mg |
| Natriumhydrogenphosphat | | 7,5 mg |
| Kochsalz | | 4,6 mg |
| Ampullenwasser | ad | 2,0 ml |

Herstellungsverfahren

Die Wirksubstanz, Puffersubstanzen und Kochsalz werden in Ampullenwasser gelöst und anschließend keimfrei filtriert.

| | |
|---|---|
| Abfüllung: | in braune Ampullen zu 2 ml unter Schutzbegasung ($N_2$) |
| Sterilisation: | 20 Minuten bei 120° C |

## Beispiel 5

Suppositorien mit 10 γ 1-(4-Äthoxycarbonylamino-3-cyan-5-fluorphenyl)-2-tert.butylamino-äthanol-hydrochlorid

Zusammensetzung:

1 Zäpfchen enthält:

| | | |
|---|---|---|
| Wirksubstanz | | 0,01 mg |
| Suppositorienmasse (z. B. Witepsol W 45) | ad | 1700,00 mg |

Herstellungsverfahren

In die geschmolzene und auf 40°C abgekühlte Zäpfchenmasse wird die feinpulverisierte Wirksubstanz mit Hilfe eines Eintauchhomogenisators eingerührt und die Masse bei 37°C in leicht vorgekühlte Formen ausgegossen.

Zäpfchengewicht: 1,7 g

Beispiel 6

Sirup mit 10 γ 1-(4-Äthoxycarbonylamino-3-cyan-5-fluor-phenyl)-2-tert.butylamino-äthanol-hydrochlorid pro 10 ml

Zusammensetzung:

100 ml Sirup enthalten:

| | | |
|---|---|---|
| Wirksubstanz | | 0,0001 g |
| Benzoesäure | | 0,1 g |
| Weinsäure | | 1,0 g |
| Zucker | | 50,0 g |
| Apfelsinen-Aroma | | 1,0 g |
| Lebensmittelrot | | 0,05 g |
| Dest. Wasser | ad | 100,0 ml |

Herstellungsverfahren

Ca. 60 g dest. Wasser werden auf 80°C erwärmt und darin nacheinander Benzoesäure, Weinsäure, die Wirksubstanz, der Farbstoff und Zucker gelöst. Nach Abkühlung auf Raumtemperatur wird das Aroma zugegeben und auf das gegebene Volumen aufgefüllt. Der Sirup wird filtriert.

Beispiel 7

Dragées mit 25 γ 1-(4-Äthoxycarbonylamino-3-cyan-5-fluor-phenyl)-2-tert.butylamino-äthanol-hydrochlorid

Zusammensetzung:

1 Dragéekern enthält:

| | |
|---|---|
| Wirksubstanz | 0,025 mg |
| Milchzucker | 82,475 mg |
| Kartoffelstärke | 33,000 mg |
| Polyvinylpyrrolidon | 4,000 mg |
| Magnesiumstearat | 0,500 mg |
| | 120,000 mg |

Herstellungsverfahren

Analog Beispiel 1.

Beispiel 8

Ampullen mit 20 γ 1-(4-Äthoxycarbonylamino-3-cyan-5-fluor-phenyl)-2-tert.butylamino-äthanol-hydrochlorid

Zusammensetzung:

1 Ampulle enthält:

| | |
|---|---|
| Wirksubstanz | 0,02 mg |
| Zitronensäure | 2,5 mg |

| | | |
|---|---|---|
| Natriumhydrogenphosphat | | 7,5 mg |
| Kochsalz | | 4,6 mg |
| Ampullenwasser | ad | 2,0 ml |

Herstellungsverfahren

Analog Beispiel 4.

**Patentansprüche**

1. Phenyläthanolamine der allgemeinen Formel I b

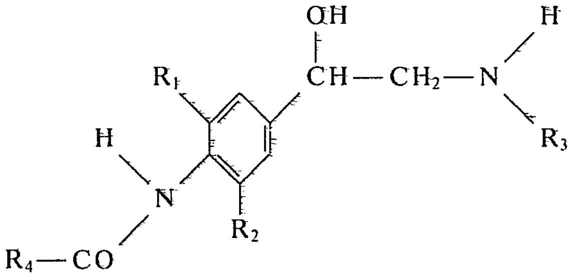

(I b)

in der

$R_1$ ein Wasserstoff-, Fluor-, Chlor-, Brom- oder Jodatom oder die Cyangruppe,
$R_2$ ein Fluoratom, die Cyan-, Trifluormethyl- oder Nitrogruppe oder eine Alkylgruppe mit 1 bis 4 Kohlenstoffatomen,
$R_3$ eine Cycloalkyl- oder Alkylgruppe mit jeweils 3 bis 5 Kohlenstoffatomen und
$R_4$ eine Alkoxygruppe mit 1 bis 5 Kohlenstoffatomen, eine Alkenyloxygruppe mit 2 bis 5 Kohlenstoffatomen, eine Aryloxygruppe mit 6 bis 10 Kohlenstoffatomen, eine Aralkoxygruppe mit 7 bis 11 Kohlenstoffatomen oder eine Aminogruppe, die durch eine Alkylgruppe mit 1 bis 5 Kohlenstoffatomen, eine Alkenylgruppe mit 2 bis 5 Kohlenstoffatomen, eine Arylgruppe mit 6 bis 10 Kohlenstoffatomen und/oder eine Aralkylgruppe mit 7 bis 11 Kohlenstoffatomen mono- oder disubstituiert sein kann, bedeuten, und deren physiologisch verträgliche Säureadditionssalze zur Verwendung bei der Bekämpfung von entzündlichen Zuständen.

2. 1-(4-Äthoxycarbonylamino-3-cyan-5-fluor-phenyl)-2-tert.butyl-amino-äthanol und dessen physiologisch verträgliche Säureadditionssalze zur Verwendung bei der Bekämpfung von entzündlichen Zuständen.

3. Verbindungen gemäß den Ansprüchen 1 und 2 zur Verwendung bei der Herstellung eines Arzneimittels mit antiphlogistischen Eigenschaften auf nichtchemischem Wege.

**Claims**

1. Phenylethanolamines of general formula Ib

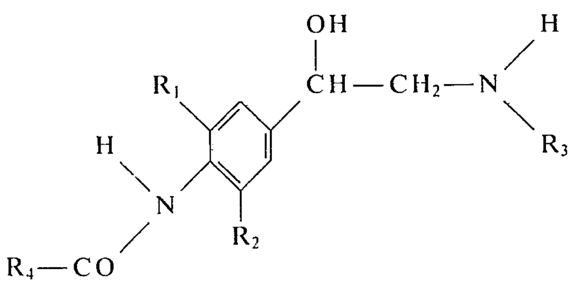

(Ib)

wherein

$R_1$ represents a hydrogen, fluorine, chlorine, bromine or iodine atom or the cyano group,
$R_2$ represents a fluorine atom, the cyano, trifluoromethyl or nitro group or an alkyl group with 1 to 4 carbon atoms,

$R_3$ represents a cycloalkyl or alkyl group with 3 to 5 carbon atoms, and
$R_4$ represents an alkoxy group with 1 to 5 carbon atoms, an alkenyloxy group with 2 to 5 carbon atoms, an aryloxy group with 6 to 10 carbon atoms, an aralkyloxy group with 7 to 11 carbon atoms or an amino group, which may be monoor disubstituted by an alkyl group with 1 to 5 carbon atoms, an alkenyl group with 2 to 5 carbon atoms, an aryl group with 6 to 10 carbon atoms and/or an aralkyl group with 7 to 11 carbon atoms, and the physiologically acceptable acid addition salts thereof for use in combating inflammatory conditions.

2. 1-(4-Ethoxycarbonylamino-3-cyano-5-fluorophenyl)-2-tert.butyl-amino-ethanol and the physiologically acceptable acid addition salts thereof for use in combating inflammatory conditions.
3. Compounds according to claims 1 and 2, for use in the preparation, by a non-chemical method, of a medicament with antiphlogistic properties.

## Revendications

1. Phényléthanolamines de formule générale Ib:

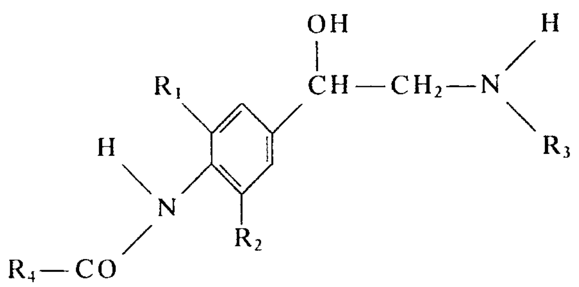

(Ib)

dans laquelle:

$R_1$ représente un atome d'hydrogène, de fluor, de chlore, de brome ou d'iode ou le groupe cyano,
$R_2$ représente un atome de fluor, le groupe cyano, trifluorométhyle ou nitro ou un groupe alcoyle avec 1 à 4 atomes de carbone,
$R_3$ représente un groupe cycloalcoyle ou alcoyle avec dans chaque cas 3 à 5 atomes de carbone, et
$R_4$ représente un groupe alcoxy avec 1 à 5 atomes de carbone, un groupe alcényloxy avec 2 à 5 atomes de carbone, un groupe aryloxy avec 6 à 10 atomes de carbone, un groupe aralcoxy avec 7 à 11 atomes de carbone, ou un groupe amino qui peut être mono- ou disubstitué par un groupe alcoyle avec 1 à 5 atomes de carbone, un groupe alcényle avec 2 à 5 atomes de carbone, un groupe aryle avec 6 à 10 atomes de carbone et/ou un groupe aralcoyle avec 7 à 11 atomes de carbone, et leurs sels d'addition avec des acides physiologiquement supportables pour l'utilisation dans la lutte contre les états inflammatoires.

2. Le 1-(4-éthoxycarbonylamino-3-cyano-5-fluorophényl)-2-tert.butylamino-éthanol et ses sels d'addition avec des acides physiologiquement supportables pour l'utilisation dans la lutte contre les états inflammatoires.
3. Composés selon les revendications 1 et 2 pour l'utilisation dans la préparation d'un médicament à propriétés antiphlogistiques par voie non chimique.